(19) Europäisches Patentamt
European Patent Office
Office européen des brevets

(11) Veröffentlichungsnummer: **0 040 761**
**A1**

(12) # EUROPÄISCHE PATENTANMELDUNG

(21) Anmeldenummer: **81103692.0**

(22) Anmeldetag: **13.05.81**

(51) Int. Cl.³: **C 07 D 233/91,** A 61 K 31/415

(30) Priorität: **23.05.80 CH 4057/80**

(43) Veröffentlichungstag der Anmeldung: **02.12.81**
**Patentblatt 81/48**

(84) Benannte Vertragsstaaten: **AT BE CH DE FR IT LI NL**

(71) Anmelder: **F. HOFFMANN-LA ROCHE & CO. Aktiengesellschaft, CH-4002 Basel (CH)**

(72) Erfinder: **Hofheinz, Werner, Dr., Talmattweg 7, CH-4103 Bottmingen (CH)**
Erfinder: **Stohler, Harro, Dr., Im Katzenwadel 12, CH-4102 Binningen (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

(54) **2-Nitroimidazole, deren Herstellung und Verwendung sowie diese enthaltende Präparate.**

(57) Die Erfindung betrifft 2-Nitroimidazole mit filaricider Wirkung der allgemeinen Formel

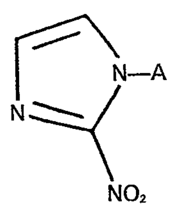

I

worin A einen der Reste $—(CH_2)_n—$ 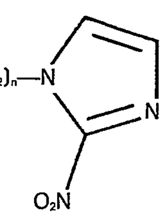

$-C(R^3)=C(R^1)(R^2)$, $-CHBr-CH_2Br$ oder $-CH_2-CCl=CH_2$ darstellt;
n 1, 2, 3 oder 4 ist;
$R^1$ und $R^2$ Wasserstoff oder $C_{1-3}$-Alkyl sind und
$R^3$ Wasserstoff, $C_{1-3}$-Alkyl, Chlor oder Brom bedeutet,

deren Herstellung in an sich bekannter Weise und deren Verwendung als Arzneimittel.

Die Erfindung betrifft ferner die Verwendung der bekannten 2-Nitroimidazole der Formel

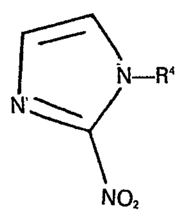

VII

worin $R^4$ $-CH_2-CH_2OH$,
$-CH_2-CHOH-CH_2OCH_3$,
$-CH_2-CHOH-CH_2OH$ oder
$-CH_2-CONH-CH_2-$ 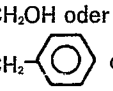 darstellt,

als filaricide Mittel.



EP 0 040 761 A1

F. Hoffmann-La Roche & Co. Aktiengesellschaft, Basel, Schweiz

RAN 4472/4

# 2-Nitroimidazole, deren Herstellung und Verwendung sowie diese enthaltende Präparate.

Die vorliegende Erfindung betrifft neue 2-Nitroimidazole der allgemeinen Formel

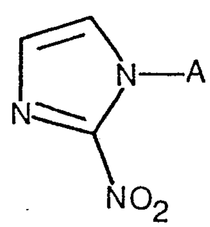

I

worin A einen der Reste 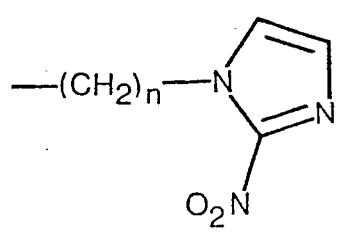 ,

$-C(R^3)=C(R^1)(R^2)$, $-CHBr-CH_2Br$ oder
$-CH_2-CCl=CH_2$ darstellt;
n 1, 2, 3 oder 4 ist;
$R^1$ und $R^2$ Wasserstoff oder $C_{1-3}$-Alkyl sind und

Mez/18.2.1981

$R^3$ Wasserstoff, $C_{1-3}$-Alkyl, Chlor oder Brom bedeutet,

deren Herstellung, deren Verwendung als Arzneimittel, insbesondere als filaricide Mittel, sowie Arzneimittel, insbesondere filaricide Mittel, auf der Basis einer Verbindung der Formel I.

Die Erfindung betrifft ferner die Verwendung der bekannten 2-Nitroimidazole der Formel

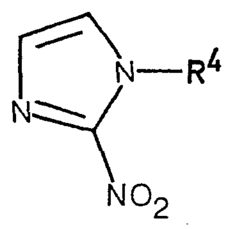

VII

worin $R^4$ $-CH_2-CH_2OH$,
$-CH_2-CHOH-CH_2OCH_3$,
$-CH_2-CHOH-CH_2OH$ oder
$-CH_2-CONH-CH_2-$ 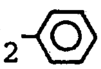 darstellt,

als filaricide Mittel und filaricide Mittel auf der Basis einer Verbindung VII.

Filarien sind fadenförmige Wurmparasiten und Verursacher verschiedener, insbesondere in Afrika, Mittel- und Südamerika, Indien, China und Südostasien weitverbreiteter Krankheiten, deren verschiedene Erscheinungsformen unter dem Begriff der Filariosen zusammengefasst werden und an denen weltweit viele Millionen Menschen leiden.

Für die Chemotherapie der Filariosen stehen bisher nur sehr wenige Mittel zur Verfügung, praktisch nur Suramin, mit Wirkung auf die adulten Filarien (Makrofilarien) und Diäthylcarbamazin, mit Wirkung auf die Filarienlarven (Mikrofilarien). Obgleich diese Medikamente aber keine optimalen Chemotherapeutika gegen diese Infektionskrankheiten darstellen (so zeigen die beiden vorstehend genannten Medikamente eine Reihe unerwünschter und teilweise gefährlicher Nebenwirkungen, wie z.B. Nierentoxizität oder allergische Reaktionen), sind in den letzten 30 bis 40 Jahren keine Fortschritte in der Chemotherapie dieser Krankheiten erzielt worden und seit 25 Jahren keine neuen Medikamente auf diesem Gebiet mehr entwickelt worden, so dass die Filariosen wahrscheinlich diejenige Gruppe von Tropenkrankheiten darstellen, gegen die neue Medikamente am dringendsten benötigt werden.

Es wurde nun gefunden, dass die neuen 2-Nitroimidazole der Formel I für die Chemotherapie von Filariosen geeignet sind und demgemäss als Arzneimittel verwendet werden können.

Ferner hat sich herausgestellt, dass die bereits bekannten Verbindungen der Formel VII auch für die Chemotherapie von Filariosen in Betracht kommen können.

Die Formel I umfasst die beiden folgenden Verbindungstypen:

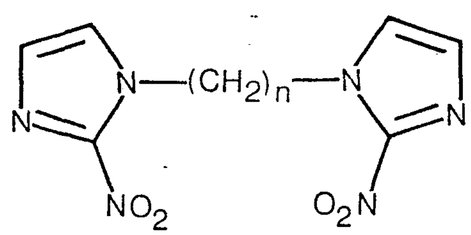

Ia

worin n 1, 2, 3 oder 4 darstellt,

und

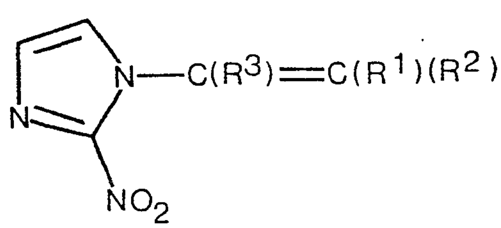

Ib

worin $R^1$ und $R^2$ Wasserstoff oder $C_{1-3}$-Alkyl und $R^3$ Wasserstoff, $C_{1-3}$-Alkyl, Chlor oder Brom

 

darstellen,
sowie 1-(1,2-Dibromethyl)-2-nitroimidazol und 1-(2-Chlorallyl)-2-nitroimidazol.

Bevorzugte Verbindungen der Formel I sind 1,2-Ethylen-bis-(2-nitroimidazol) und 2-Nitro-1-vinylimidazol.

Die erfindungsgemässen 2-Nitroimidazole der Formel I können in an sich bekannter Weise hergestellt werden und zwar beispielsweise dadurch, dass man entweder

a) Azomycin mit einem disubstituierten aliphatischen Kohlenwasserstoff der Formel $Y-(CH_2)_n-Y$ oder $Y-CH_2-CCl=CH_2$, worin Y Halogen oder eine andere abspaltbare Gruppe darstellt, umsetzt oder

b) ein 2-Nitroimidazol der Formel

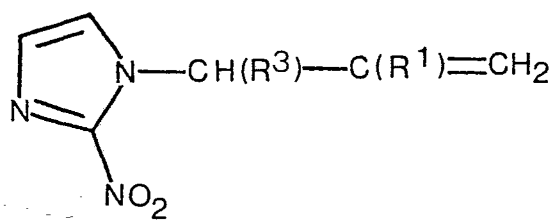

II

worin $R^1$ und $R^3$ wie oben definiert sind, isomerisiert oder

c) aus einem 2-Nitroimidazol der Formeln

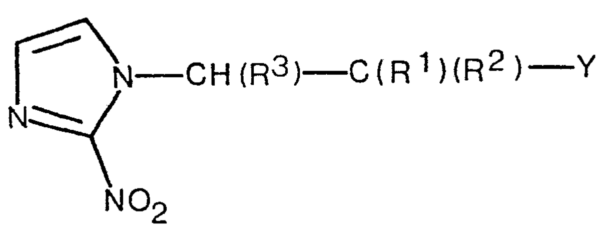

III

oder

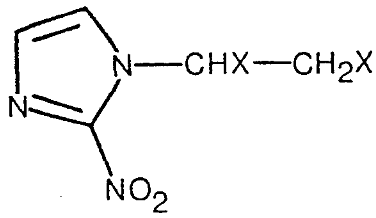 IV

worin $R^1$, $R^3$ und Y wie oben definiert sind und X Chlor oder Brom darstellt,

HX bzw. HY abspaltet oder

d) an 2-Nitro-1-vinylimidazol Brom anlagert.

Gemäss Verfahrensvariante a) wird Azomycin mit einem disubstituierten Alkan der Formel $Y-(CH_2)_n-Y$ oder mit $Y-CH_2-CCl=CH_2$, worin Y Halogen oder eine abspaltbare Gruppe, vorzugsweise Chlor, Brom, Jod oder Tosyloxy bedeutet, umgesetzt. In einer Verbindung $Y-(CH_2)_n-Y$ können die beiden Reste Y gleich oder voneinander verschieden sein. Die Umsetzung kann in einem der üblichen inerten organischen Lösungsmittel, wie Dimethylformamid, Dimethylacetamid oder Methylenchlorid, bei Temperaturen zwischen Raumtemperatur und der Rückflusstemperatur des Reaktionsgemisches, gegebenenfalls in Gegenwart einer Base durchgeführt werden. Als Reaktionsprodukte erhält man 1-(2-Chlorallyl)-2-nitroimidazol und, in Abhängigkeit von dem Mengenverhältnis der eingesetzten Ausgangsmaterialien Azomycin und $Y-(CH_2)_n-Y$, Bis-(2-nitroimidazole) der Formel Ia und/oder Verbindungen der Formel

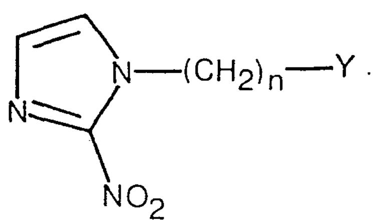 V

Je grösser der Ueberschuss an Azomycin ist, dester höher ist der Anteil an Verbindungen Ia im Reaktionsgemisch. Die Gemische aus Verbindungen Ia und V können auf einfache Weise, z.B. auf Grund ihrer unterschiedlichen Löslichkeiten, aufgetrennt werden (vgl. Beispiel 2). Eine Verbindung der Formel V kann durch weitere Umsetzung mit Azomycin in eine Verbindung Ia übergeführt werden.

Gemäss Verfahrensvariante b) kann eine Verbindung der Formel II zu einer Verbindung der Formel

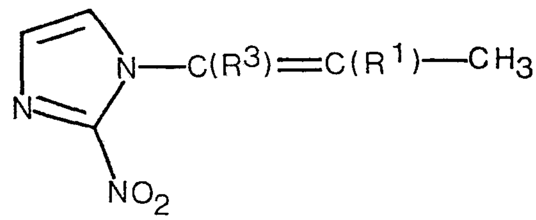
Ic

isomerisiert werden. Dies kann z.B. durch Behandlung mit Schwefelsäure erfolgen. Die Temperatur ist nicht kritisch, sodass vorzugsweise bei Raumtemperatur gearbeitet wird.

Gemäss Verfahrensvariante c) erhält man durch Abspaltung von HY bzw. HX aus Verbindungen der Formeln III oder IV Verbindungen der allgemeinen Formeln

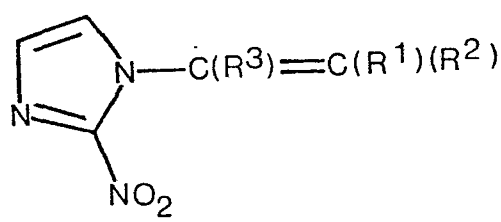
Ib

bzw.

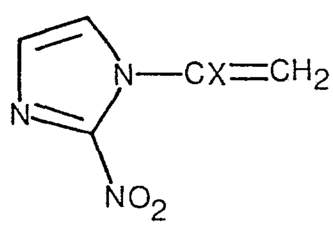
Id

Die Eliminierung von HX bzw. HY kann in an sich bekannter Weise, z.B., insbesondere wenn Y Halogen oder Tosyloxy darstellt, durch Behandlung mit einem Alkalimetallalkoholat

in einem inerten organischen Lösungsmittel, vorzugsweise einem Alkanol, erfolgen. Ist die abspaltbare Gruppe Y in einer Verbindung III Hydroxy, so wird sie zweckmässigerweise zunächst in eine Tosyloxygruppe übergeführt. Eine Verbindung III mit Y = Hydroxy kann aber auch direkt durch Behandlung mit einer starken Säure (Sulfonsäure, Trifluoressigsäure, Schwefelsäure) in eine Verbindung Ib übergeführt werden.

Durch Anlagerung von Brom an 2-Nitro-1-vinylimidazol gemäss Verfahrensvariante d) erhält man 1-(1,2-Dibromäthyl)-2-nitroimidazol. Man arbeitet zweckmässigerweise in einem inerten organischen Lösungsmittel, z.B. in Dichlormethan, und unter Eiskühlung.

Die Ausgangsverbindung der Formel II kann durch Umsetzung von Azomycin mit einer Verbindung $Y-CH(R^3)-C(R^1)=CH_2$ oder durch Abspaltung von HY aus einer Verbindung

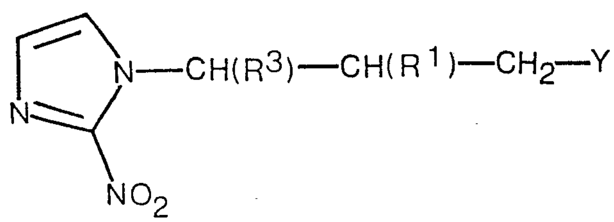

VI

erhalten werden. Die Herstellung der Verbindung VI kann in Analogie zu der unter Variante a) beschriebenen Methode für die Herstellung einer Verbindung V erfolgen, die Abspaltung von HY aus einer Verbindung VI in Analogie zu den unter Variante c) beschriebenen Methoden.

Die erfindungsgemässen Verbindungen zeigen mikro- und insbesondere makro-filaricide Wirkung. Beispielsweise betragen für 1,2-Aethylen-bis-(2-nitroimidazol) und für 2-Nitro-1-vinylimidazol die an der Baumwollratte gegen Litomosoides carinii ermittelten $ED_{90}$-Werte 147 mg/kg bzw. 12 mg/kg (macrofilaricide Aktivität). Sie können demgemäss als Arzneimittel, insbesonders als Filaricide, in Form pharmazeutischer Präparate in Mischung mit einem geeigneten

organischen oder anorganischen inerten Trägermaterial, z.B. Wasser, Gelatine, Gummi arabicum, Milchzucker, Stärke, Magnesiumstearat, Talk, pflanzlichen Oelen oder Polyalkylenglykolen, verwendet werden. Die pharmazeutischen Präparate können in fester Form, z.B. als Tabletten, Dragées oder Kapseln oder in flüssiger Form, z.B. als Lösungen, Suspensionen oder Emulsionen, vorliegen. Gegebenenfalls sind sie sterilisiert und bzw. oder enthalten weitere Hilfsstoffe, wie Konservierungs-, Stabilisierungs-, Netz- oder Emulgiermittel, Mittel zur geschmacklichen Verbesserung, Salze zur Veränderung des osmotischen Druckes oder Puffersubstanzen. Die Herstellung der pharmazeutischen Präparate kann in der jedem Fachmann geläufigen Weise erfolgen.

Die Applikationsform der Wahl ist die orale. Als Richtlinie für den Dosierungsbereich - ohne dass damit eine Limitierung ausgesprochen sein soll - kann, in Abhängigkeit von der Art und Schwere der Infektion einerseits und der Aktivität der zu verabreichenden Wirksubstanz andererseits, eine Menge von ca. 5-150 mg/kg Körpergewicht pro Tag, verabreicht in ein oder mehreren Einzeldosen und während bis zu einer Woche, gelten.

## Beispiel 1

56 g Azomycin und 84 g Natriumbicarbonat wurden in 300 ml Dimethylformamid und 47 g 1,2-Dibromethan während 6 Stunden unter Rückfluss gekocht. Es wurde auf 1500 ml Eiswasser gegossen und der Niederschlag abfiltriert. Nach Umkristallisieren aus 650 ml Nitromethan erhielt man 36 g 1,1'-Ethylen-bis-(2-nitroimidazol), Smp. 248-250°C (Zers.)

## Beispiel 2

206 g 1-(2-Jodethyl)-2-nitroimidazol wurden in einer Lösung von 41 g Natriummethylat in 2 l Methanol 1 Stunde lang unter Rückfluss gekocht. Die Lösung wurde unter vermindertem Druck eingedampft und der Rückstand mit 2 l Essigester und 1 l Wasser aufgenommen. Die organische Phase wurde zweimal mit je 0,5 l 10%iger Kochsalzlösung gewaschen und zur Trockene eingedampft. Der Rückstand lieferte nach Umkristallisation aus Diisopropyläther 98,2 g 2-Nitro-1-vinyl-imidazol, Smp. 64-66°C.

Das Ausgangsmaterial wurde folgendermassen erhalten:

340 g Azomycin und 510 g Natriumbicarbonat wurden in 2000 ml Dimethylformamid und 1200 g 1,2-Dichlor-ethan 4 Stunden lang bei 100°C gerührt. Nach Abkühlen wurde filtriert, der grösste Teil des Lösungsmittels im Wasserstrahlvakuum bei einer Badtemperatur von ca. 60°C abgezogen und der Rückstand mit 4 l Essigester verrührt. 1,1'-Ethylen-bis-(2-nitroimidazol) fiel als schwerlösliches kristallines Produkt aus, das abfiltriert und aus 700 ml Nitromethan umkristallisiert wurde; Ausbeute 56,5 g; Smp. 248-250°C (Zers.).

Das Filtrat wurde dreimal mit je 1 l 10%iger Kochsalzlösung gewaschen und unter vermindertem Druck eingedampft. Nach Kristallisation aus 1 l Essigester erhielt man 395 g 1-(2-Chlorethyl)-2-nitroimidazol, Smp. 85-87°C.

163,4 g 1-(2-Chlorethyl)-2-nitroimidazol und 200,4 g Natriumjodid wurden in 1 l Ethylmethylketon 4 1/2 Stunden unter Rückfluss erhitzt. Nach dem Abkühlen wurde filtriert, das Lösungsmittel abgedampft, der Rückstand zwischen 2 l Dichlormethan und 1 l Wasser verteilt und die organische Phase zweimal mit je 1 l Wasser gewaschen und eingedampft. Kristallisation aus 1 l Isopropanol lieferte 206 g 1-(2-Jodethyl)-2-nitroimidazol, Smp. 79-82°C.

## Beispiel 3

Zu einer Lösung von 69,5 g 2-(2-Nitro-1-imidazolyl)-ethanol in 400 ml Pyridin wurde portionenweise, unter Eiskühlung, 92 g p-Toluolsulfochlorid zugegeben. Nach Stehen über Nacht bei Raumtemperatur wurde auf ein Gemisch aus 750 g Eis und 250 ml konz. Salzsäure gegossen. Das ausgefällte Tosylat wurde abgenutscht und aus Isopropanol umkristallisiert; Ausbeute 73 g; Smp. 103-105°C.

31,3 g des Tosylats wurden in 300 ml Toluol gelöst und unter Eiskühlung mit 51,2 ml einer 2,15 N Lösung von Natrium-tert.-amylat in Toluol behandelt. Dann wurde auf 300 ml 0,7 N Salzsäure gegossen, die wässrige Phase abgetrennt, die organische Phase mit 10%iger Kochsalzlösung gewaschen und unter vermindertem Druck zur Trockene eingeengt. Der Rückstand, aus Isopropyläther umkristallisiert, lieferte 9,5 g 2-Nitro-1-vinylimidazol, Smp. 62-63°C.

## Beispiel 4

21,8 g Azomycin in 120 ml Dimethylformamid wurden während 5 Stunden mit 32,5 g Natriumbicarbonat und 17,5 g Methallylchlorid bei 100°C umgesetzt. Anschliessend wurde auf 1 l Wasser gegossen und das ausgefällte 1-(2-Methylallyl)-2-nitroimidazol abgenutscht; Ausbeute 12,1 g; Smp. 49-51°C. Durch Extraktion des Filtrats mit Essigester konnten weitere 13,8 g der Verbindung gewonnen werden.

23,8 g 1-(2-Methylallyl)-2-nitroimidazol wurden in 110 ml konz. Schwefelsäure bei Raumtemperatur gelöst. Nach 30 Minuten wurde auf 1 l Eis/Wasser gegossen, mit konz. wässrigem Ammoniak neutralisiert und abgenutscht. Umkristallisation aus Diisopropyläther lieferte 5,4 g 1-(2-Methylpropenyl)-2-nitroimidazol, Smp. 77-79°C.

Beispiel 5

20,1 g 1-(1,2-Dibromethyl)-2-nitroimidazol wurden in 132 ml einer 0,51 N Lösung von Natriumethylat in Methanol eine Stunde unter Rückfluss erhitzt. Das Gemisch wurde eingedampft und der Rückstand mit 250 ml Essigester und 100 ml Wasser aufgenommen. Die organische Phase wurde mit 100 ml 10%iger Kochsalzlösung gewaschen und eingedampft und der Rückstand mittels Essigester-Cyclohexan (4:1, v/v) an 300 g Kieselgel chromatographiert. Nach einem Vorlauf von 420 ml wurden aus 270 ml weiterem Eluat 8,2 g 1-(1-Bromvinyl)-2-nitroimidazol isoliert; Ausbeute 4,6 g; Smp. 80-82°C (aus Isopropanol).

Beispiel 6

Zu einer Lösung von 21 g 2-Nitro-1-vinylimidazol in 150 ml Dichlormethan wurde unter Rühren und Eiskühlung eine Lösung von 24 g Brom in 75 ml Dichlormethan getropft. Nach 5 Stunden wurde filtriert und das Filtrat am Rotationsverdampfer zur Trockene eingedampft. Der Rückstand, aus Ethanol-Wasser unter Zusatz von Aktivkohle umkristallisiert, lieferte 34 g 1-(1,2-Dibromethyl)-2-nitroimidazol, Smp. 102-106°C.

## Beispiel 7

Tablette enthaltend

| | |
|---|---|
| 1,2-Ethylen-bis-(2-nitroimidazol) | 100 mg |
| Lactose | 192 mg |
| Maisstärke | 80 mg |
| Hydrolysierte Maisstärke | 20 mg |
| Calciumstearat | 8 mg |
| | 400 mg |

## Beispiel 8

Tablette enthaltend

| | |
|---|---|
| 2-Nitro-1-vinylimidazol | 50 mg |
| Lactose | 194 mg |
| Vorgelatinierte Maisstärke | 150 mg |
| Calciumstearat | 6 mg |
| | 400 mg |

## Patentansprüche

1. 2-Nitroimidazole der allgemeinen Formel

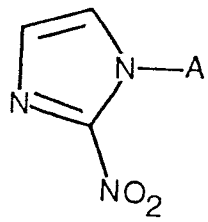

I

worin A einen der Reste 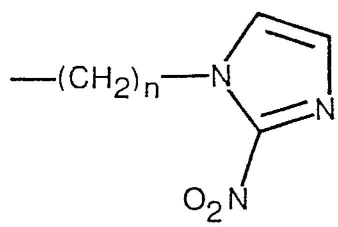 ,

$-C(R^3)=C(R^1)(R^2)$, $-CHBr-CH_2Br$ oder $-CH_2-CCl=CH_2$ darstellt;

n 1, 2, 3 oder 4 ist;

$R^1$ und $R^2$ Wasserstoff oder $C_{1-3}$-Alkyl sind und

$R^3$ Wasserstoff, $C_{1-3}$-Alkyl, Chlor oder Brom bedeutet.

2. 2-Nitroimidazole der allgemeinen Formel

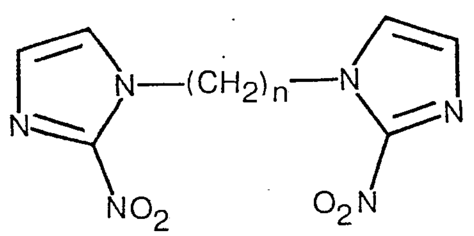

Ia

worin n 1, 2, 3 oder 4 bedeutet.

3. 1,2-Ethylen-bis-(2-nitroimidazol).

4. 2-Nitroimidazole der allgemeinen Formel

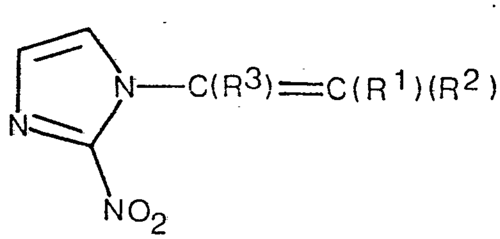

Ib

worin $R^1$ und $R^2$ Wasserstoff oder $C_{1-3}$-Alkyl und $R^3$ Wasserstoff, $C_{1-3}$-Alkyl, Chlor oder Brom darstellen.

5. 2-Nitro-1-vinylimidazol.

6. 2-Nitroimidazole gemäss einem der Ansprüche 1-5 als pharmazeutische Wirkstoffe.

7. 2-Nitroimidazole gemäss einem der Ansprüche 1-5 als filaricide Mittel.

8. 2-Nitroimidazole der Formel

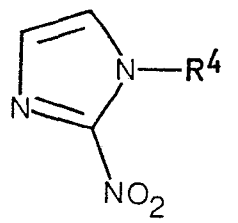

VII

worin $R^4$ - $CH_2$-$CH_2OH$,
- $CH_2$-CHOH- $CH_2OCH_3$,
- $CH_2$-CHOH-$CH_2OH$ oder

- $CH_2$-CONH-$CH_2$- 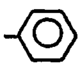 darstellt,

als filaricide Mittel.

9. Verfahren zur Herstellung von 2-Nitroimidazolen der allgemeinen Formel

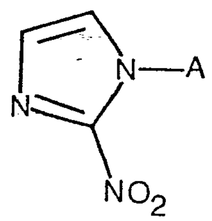 I

worin A einen der Reste 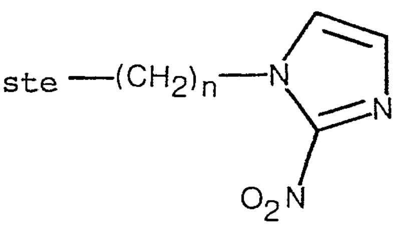 ,

$-C(R^3)=C(R^1)(R^2)$, $-CHBr-CH_2Br$ oder $-CH_2-CCl=CH_2$ darstellt;

n 1, 2, 3 oder 4 ist;

$R^1$ und $R^2$ Wasserstoff oder $C_{1-3}$-Alkyl sind und

$R^3$ Wasserstoff, $C_{1-3}$-Alkyl, Chlor oder Brom bedeutet,

dadurch gekennzeichnet, dass man entweder

a) Azomycin mit einem disubstituierten aliphatischen Kohlenwasserstoff der Formel $Y-(CH_2)_n-Y$ oder $Y-CH_2-CCl=CH_2$, worin Y Halogen oder eine abspaltbare Gruppe darstellt, umsetzt oder

b) ein 2-Nitroimidazol der Formel

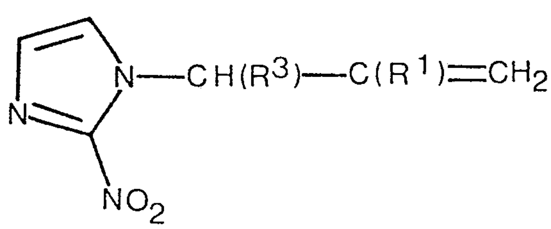 II

worin $R^1$ und $R^3$ wie oben definiert sind, isomerisiert oder

c) aus einem 2-Nitroimidazol der Formeln

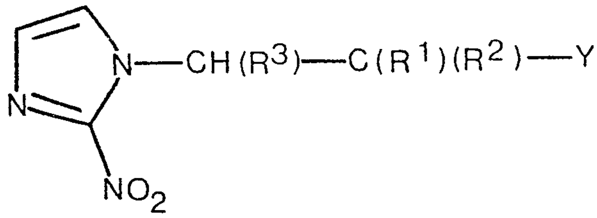

III

oder

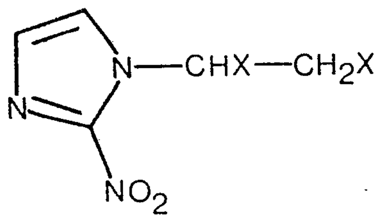

IV

worin $R^1$, $R^3$ und Y wie oben definiert sind und X Chlor oder Brom darstellt,

HX bzw. HY abspaltet oder

d) an 2-Nitro-1-vinylimidazol Brom anlagert.

10. Arzneimittel enthaltend ein 2-Nitroimidazol gemäss einem der Ansprüche 1-5.

11. Filaricides Mittel enthaltend ein 2-Nitroimidazol gemäss einem der Ansprüche 1-5.

12. Filaricides Mittel enthaltend ein 2-Nitroimidazol der Formel VII gemäss Anspruch 8.

13. Verwendung von 2-Nitroimidazolen gemäss den Ansprüchen 1-5 bei der Bekämpfung von Krankheiten.

14. Verwendung von 2-Nitroimidazolen gemäss den Ansprüchen 1-5 bei der Bekämpfung von Filariosen.

15. Verwendung von 2-Nitroimidazolen der Formel VII gemäss Anspruch 8 bei der Bekämpfung von Filariosen.

***

## Patentanspruch
"für OESTERREICH"

Verfahren zur Herstellung von 2-Nitroimidazolen der allgemeinen Formel

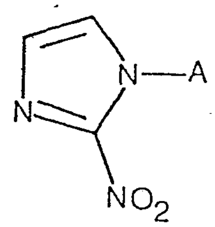

I

worin A einen der Reste 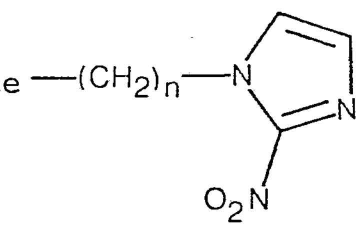 ,

$-C(R^3)=C(R^1)(R^2)$, $-CHBr-CH_2Br$ oder $-CH_2-CCl=CH_2$ darstellt;
n 1, 2, 3 oder 4 ist;
$R^1$ und $R^2$ Wasserstoff oder $C_{1-3}$-Alkyl sind und
$R^3$ Wasserstoff, $C_{1-3}$-Alkyl, Chlor oder Brom bedeutet,

dadurch gekennzeichnet, dass man entweder

a) Azomycin mit einem disubstituierten aliphatischen Kohlenwasserstoff der Formel $Y-(CH_2)_n-Y$ oder $Y-CH_2-CCl=CH_2$, worin Y Halogen oder eine abspaltbare Gruppe darstellt, umsetzt oder

b) ein 2-Nitroimidazol der Formel

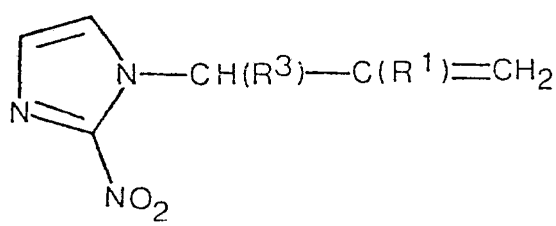

II

worin $R^1$ und $R^3$ wie oben definiert sind, isomerisiert oder

c) aus einem 2-Nitroimidazol der Formeln

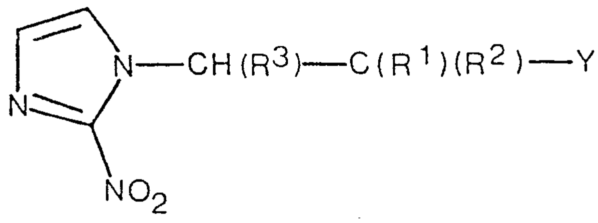

III

oder

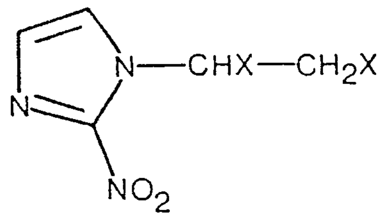

IV

worin $R^1$, $R^3$ und Y wie oben definiert sind und X Chlor oder Brom darstellt,

HX bzw. HY abspaltet oder

d) an 2-Nitro-1-vinylimidazol Brom anlagert.

***

0040761

Nummer der Anmeldung

Europäisches Patentamt

# EUROPÄISCHER RECHERCHENBERICHT

EP 81 10 3692.0

| Kategorie | EINSCHLÄGIGE DOKUMENTE — Kennzeichnung des Dokuments mit Angabe, soweit erforderlich, der maßgeblichen Teile | betrifft Anspruch |
|---|---|---|
| X | US - A - 3 391 156 (A.G. BEAMAN et al.) * Ansprüche; Spalte 1, Zeile 45; Spalte 2, Zeilen 18 bis 30 * | 1,4-6, 10, 11, 13-15 |
| | US - A - 3 641 047 (A.G. BEAMAN et al.) * Verbindungen Spalte 1; Spalte 4, Zeilen 16 bis 26 * | 8,12, 15 |
| | US - A - 3 679 698 (A.G. BEAMAN et al.) * Beispiel 6; Spalte 5, Zeilen 38 bis 47 * | 8,12, 15 |
| | DE - A - 1 695 131 (F. HOFFMANN-LA ROCHE & CO.) * Verbindungen der Formel I * | 8 |

**KLASSIFIKATION DER ANMELDUNG (Int. Cl.³)**

C 07 D 233/91
A 61 K 31/415

**RECHERCHIERTE SACHGEBIETE (Int. Cl.³)**

A 61 K 31/415
C 07 D 233/91

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 27-08-1981 | FROELICH |